# EUROPEAN PATENT SPECIFICATION

(11) **EP 2 409 987 B1**
(45) Date of publication and mention of the grant of the patent: **26.06.2013**
(21) Application number: 11167994.0
(22) Date of filing: 27.05.2011
(51) Int. Cl.: C07K 14/445, A61K 39/015

(54) **Ribosomal P protein complex from Plasmodium falciparum as antigen in vaccines against malaria**
Ribosomaler P Protein Komplex von Plasmodium falciparum als Antigen in Impfstoffen gegen Malaria
"Ribosomal P protein complex" de Plasmodium falciparum comme antigene d'un vaccin contre la malaria

(30) Priority: 31.05.2010 PL 39138410
(43) Date of publication of application: 25.01.2012
(73) Proprietor: Uniwersytet Marii Curie Sklodowskiej, 20-031 Lublin (PL)
(72) Inventor: Tchórzewski, Marek, 20-801 Lublin (PL); Krokowski, Dawid, 34-500 Zakopane (PL); Szuster-Ciesielska, Agnieszka, 20-616 Lublin (PL); Grankowski, Nikodem, 20-709 Lublin (PL); Wawiórka, Leszek, 20-849 Lublin (PL)
(74) Representative: Kalita, Lucjan

(56) References cited:
- EP-A1- 2 116 261
- K. RAJESHWARI ET AL: "The P Domain of the P0 Protein of Plasmodium falciparum Protects against Challenge with Malaria Parasites", INFECTION AND IMMUNITY, vol. 72, no. 9, 1 September 2004 (2004-09-01), pages 5515-5521, XP55014129, ISSN: 0019-9567, DOI: 10.1128/IAI.72.9.5515-5521.2004
- CHATTERJEE SANCHITA ET AL: "Antibodies against ribosomal phosphoprotein P0 of Plasmodium falciparum protect mice against challenge with Plasmodium yoelii", INFECTION AND IMMUNITY, AMERICAN SOCIETY FOR MICROBIOLOGY, WASHINGTON, US, vol. 68, no. 7, 1 July 2000 (2000-07-01), pages 4312-4318, XP002204978, ISSN: 0019-9567, DOI: 10.1128/IAI.68.7.4312-4318.2000
- GABRIELA AREVALO-PINZON ET AL: "Fine mapping of Plasmodium falciparum ribosomal phosphoprotein PfP0 revealed sequences with highly specific binding activity to human red blood cells", JOURNAL OF MOLECULAR MEDICINE, SPRINGER, BERLIN, DE, vol. 88, no. 1, 21 September 2009 (2009-09-21), pages 61-74, XP019771194, ISSN: 1432-1440
- SHARMA SHOBHONA ET AL: "Malaria vaccine: a current perspective", JOURNAL OF VECTOR BORNE DISEASES, vol. 45, no. 1, March 2008 (2008-03), pages 1-20, XP007919879, ISSN: 0972-9062
- SUTHERLAND ET AL: "Surface antigens of Plasmodium falciparum gametocytes-A new class of transmission-blocking vaccine targets?", MOLECULAR AND BIOCHEMICAL PARASITOLOGY, ELSEVIER SCIENCE PUBLISHERS, AMSTERDAM, NL, vol. 166, no. 2, 1 August 2009 (2009-08-01), pages 93-98, XP026193381, ISSN: 0166-6851, DOI: 10.1016/J.MOLBIOPARA.2009.03.007 [retrieved on 2009-03-28]
- MALCOLM J GARDNER ET AL: "Genome sequence of the human malaria parasite Plasmodium falciparum", NATURE: INTERNATIONAL WEEKLY JOURNAL OF SCIENCE, NATURE PUBLISHING GROUP, UNITED KINGDOM, vol. 419, no. 6906, 3 October 2002 (2002-10-03), pages 498-511, XP002622611, ISSN: 0028-0836, DOI: 10.1038/NATURE01097 & DATABASE UniProt [Online] 1 March 2003 (2003-03-01), "60S ribosomal protein P1, putative;", retrieved from EBI accession no. UNIPROT:Q8IIX0 Database accession no. Q8IIX0 & DATABASE UniProt [Online] 1 March 2003 (2003-03-01), "60S ribosomal protein P0;", retrieved from EBI accession no. UNIPROT:Q8II61 Database accession no. Q8II61 & DATABASE UniProt [Online] 1 July 1997 (1997-07-01), "60S acidic ribosomal protein P2;", retrieved from EBI accession no. UNIPROT:O00806 Database accession no. O00806

## Description

Ribosomal P protein complex from *Plasmodium falciparum* as an antigen for use in vaccine against malaria.

The subject of the invention is a pentameric P protein comlex P0(₁₉₇₋₃₁₆)-(P1-P2)₂ from *Plasmodium falciparum* demonstrating immunogenic properties, that protect organism from the clinical symptoms of malaria, and the method of obtaining the same using polycistronic expression cassette comprising three genes as the genetic expression system.

Malaria, caused by protozoa of the genus *Plasmodium,* is currently one of the most dangerous and rapidly spreading infectious diseases in tropical regions. The most severe course takes the infections caused by the species *Plasmodium falciparum*, which is highly resistant to drugs known so far. Therefore the use of vaccine containing appropriate antigen which induces a specific immunity protecting an organism against the clinical symptoms of malaria is the only effective method of tackling the disease.

Currently no effective vaccine is available, that would fully protect against the infection and clinical manifestation of the disease caused by this protozoa. This problem is related to the complicated life cycle of *Plasmodium* and the high variability of the surface antigens specific for certain life stages of the pathogenic protozoa (Waters, A. Cell 124, 687-693, 2006).

The life cycle of *Plasmodium falciparum* consists of several stages both in humans and in mosquitoes, which relate to certain antigens inducing specific immunological response in humans. The bites by mosquitoes belonging to *Anopheles* species, deliver the so called sporozoites into the blood stream and through the blood vessels they migrate to the liver and invade hepatocites, initiating the hepatic stage of the parasite life cycle. In the hepatocites the sporozoites develop into schizonts and after 7 days merozoites are released from liver to bloodstream where they infect erythrocytes. The aforementioned forms proliferate in erythrocytes, and then they metamorphose into trophozoides, that turn into a new generation of schizonts, that is the erythrocytic schizonts. Subsequently these schizonts differentiate to merozoits that further infect erythrocytes, causing rapid onset of the infection.

Taking the known strategies of vaccine design into account, the antigens from a large group of parasite antigens specific for different development stages were considered as potential components of the vaccine against malaria. So far, a number of antigens specific for different development stages have been described as potential vaccine components. However until now no effective vaccines have been devised.

During the studies carried out by GlaxoSmithKline Biologicals and Apovia Inc., San Diego the following preparations were tested: RTS, S/AS02A (Gordon D.M., J. Infect. Dis., 171, 1995) and ICC-1132 CS/hepatitis B based on *Plasmodium falciparum* CSP protein (Birkett A., Infect. Immun. 70, 2002) and they did not show satisfactory results towards effective vaccine.

Currently the MSP1 protein has been tested extensively as an antigen, however preparations, using this protein, e.g. FMP-1/AS02A devised by The Walter Reed Army Institute of Research in cooperation with other research centers (Angov E., Mol. Biochem. Parasito., 128, 2003) as well as by the University of Hawaii (Chang D.P., Infect. Immun. 64, 1996) and the Pasteur Institute (Garraud O., Scand. J. Immunol., 49, 1999) proved to be ineffective.

Similarly, the test of AMP1 protein, known as the FMP2.1 preparation devised by The Walter Reed Army Institute of Research, together with USAID and GSKBio as well as these of PfCP-2.9 chimeric protein - obtained by fusing AMP1 and MSP1 protein - and carried out by The second Military Medical University in Shanghai and World Health Organization have failed. Further studies performed by Malaria Vaccine Development Unit of National Institutes of Health USA (Ballou W.R., Am.J.Trop.Med.Hyg., 71 (Suppl 2), 2004) focused on Pfs25 protein, but again were not successful. The published data (Chatterjee S., Mol. Biochem. Parasitol., 107/2000) indicate that a single ribosomal protein P0 is immunogenic. However this single protein was only a weak inducer of the production of protective antibodies against *Plasmodium* infection. Moreover, in nature it is found in complex with P1 and P2 proteins, which excludes the possibility of using it as a single component to obtain a malaria vaccine on a large scale.

The main idea behind this invention is the lack of data concerning immunological reactions to P1 and P2 proteins, that is mainly due to their physicochemical properties, *i.e*. low molecular weight about 10 kD and low pI~4, making the analysis and preparation difficult. These observations gave authors an idea for the invention and inspired to carry out the research to obtain the antigen of malaria pathogen, having both the most advantageous immunostimulating and immunoprotecting properties.

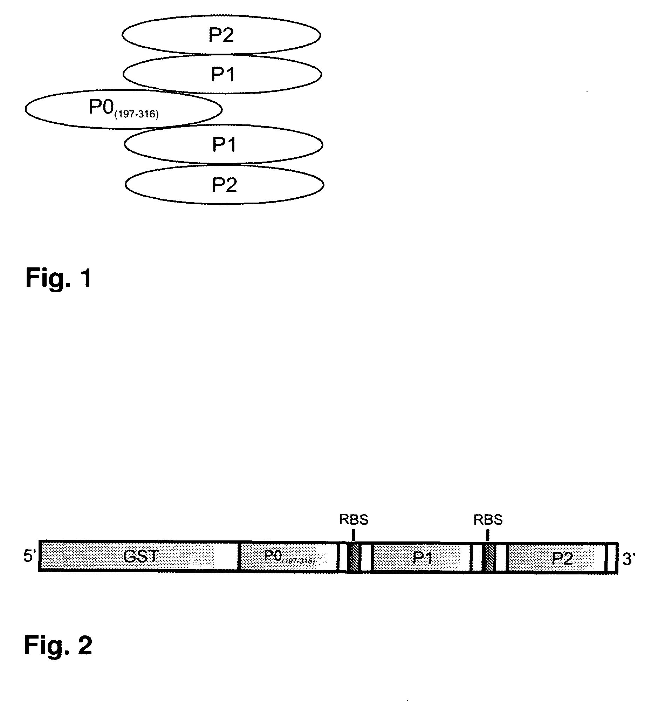

Unexpectedly, the objective was achieved by using the ribosomal P proteins complex from *Plasmodium falciparum.* The invention relates to the antigen of malaria pathogen in the pentameric form of P protein complex, described as P0₍₁₉₇₋₃₁₆₎-(P1-P2)₂, containing ribosomal P0 protein fragment including amino acid residues from 197 to 316 and two complete ribosomal P1 and P2 proteins, preferably present in heterodimeric complex shown in Fig. 1 and Sequence 1.

The antigen, according to the invention, is in soluble and in stable form, showing strong immunostimulating properties. These properties have been confirmed in biochemical and immunological studies described in Example 1 and 2.

The invention relates also to the use of the antigen defined above for obtaining of the vaccines against malaria. The invention relates also to the method of obtaining the antigen, according to the invention, including the method of protein overexpression using the vector that carries the expression cassette, containing three genes as a polycistronic system, which encodes amino acid sequences of pentameric P protein complex components.

The invention relates also to the expression cassette to be used according to the invention and the expression sequence is the system of three genes positioned in P0-P1-P2 sequence, of which every gene contains the separate regulatory RBS sequences and the linker sequences, and preferably the gene coding P0 protein fragment is fused to gene coding GST protein. The expression cassette according to the invention has been presented as Sequence 2, described below (Example 4) and visualized as schematic diagram in Fig. 2.

The cassette comprises the gene encoding GST protein fused to the gene encoding P0₍₁₉₇₋₃₁₆₎ protein fragment and genes for P1 and P2 proteins. RBS - regulatory sequence responsible for efficient initiation of translation. 5' and 3' indicate the DNA orientation.

### Description of the drawings

Fig. 1 shows the diagram of the pentameric P protein complex, P0₍₁₉₇₋₃₁₆₎-(P1-P2)₂.
Fig. 2 shows the expression cassette.
Fig. 3 shows the SDS-PAGE analysis of the P protein complex at the subsequent purification stages, where:
   markers - molecular mass marker proteins
   cell extract -IPTG, - no expression induced
   cell extract +IPTG, - induced expression of the P protein complex
   inclusion bodies - fraction of insoluble proteins
   S-1 00 fraction - soluble proteins fraction
   GST-P0(₁₉₇₋₃₁₆)-(P1-P2)₂ - fraction containing P proteins complex fused with GST P0(₁₉₇₋₃₁₆)-(P1-P2)₂ - the product, purified P proteins complex, devoid of GST protein.

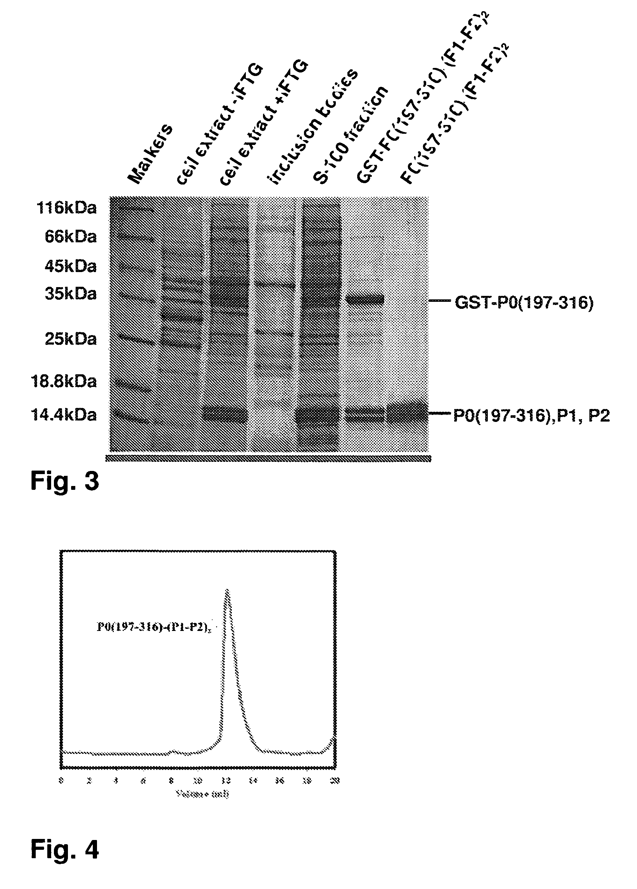

Fig. 4 shows the analysis employing size exclusion chromatography.

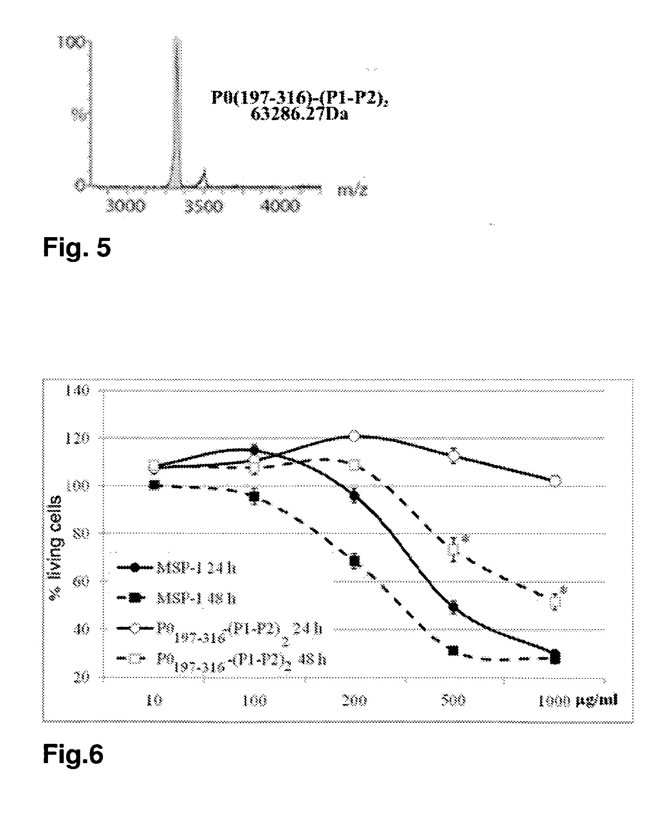

Fig. 5 shows the mass spectrometry analysis of the P protein complex under non-denaturing conditions.
Fig. 6 shows the viability of the murine spleen lymphocytes following incubation for 24 h and 48 h in the presence of P protein complex and MSP-1 protein at different concentrations. Asterisk denotes the statistically significant differences (p≤0.05), comparing to 24 h-incubation time.

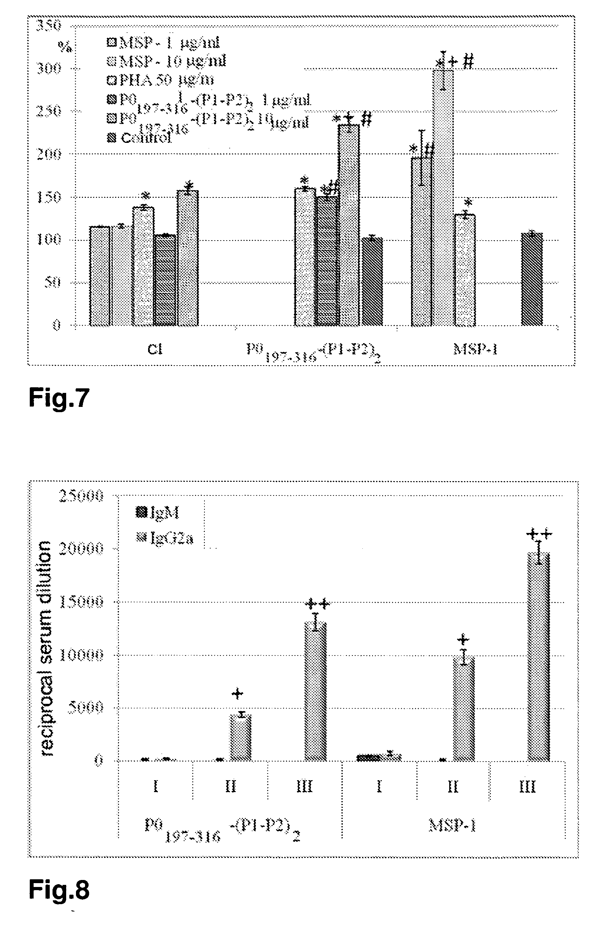

Fig. 7 shows the effect of P protein complex and MSP-1 from *P. falciparum* on the proliferation of murine spleen lymphocytes. K1 - proliferation of lymphocytes isolated from mice that received only the Freund adjuvant; P0(₁₉₇₋₃₁₆)-(P1-P2)₂, proliferation of lymphocytes isolated from mice that were immunized with P protein complex; MSP-1, proliferation of lymphocytes isolated from mice that were immunized with MSP-1 protein; Asterisk indicates statistically significant differences in comparison to the control (lymphocyte of non-immunized mice); Plus sign indicates statistically significant differences in comparison to a lower protein concentration; Hash denotes statistically significant differences in comparison to the same protein concentration applied to the corresponding control sample (lymphocytes isolated from mice that received adjuvants, PBS and mice that were not immunized, respectively).
Fig. 8 shows the levels of IgM and IgG2a - class antibodies in the sera of mice immunized with the P protein complex and MSP-1 protein. Plus sign indicates statistically significant differences in comparison to immunization I; Double plus sign denotes statistically significant differences in comparison to immunization II;

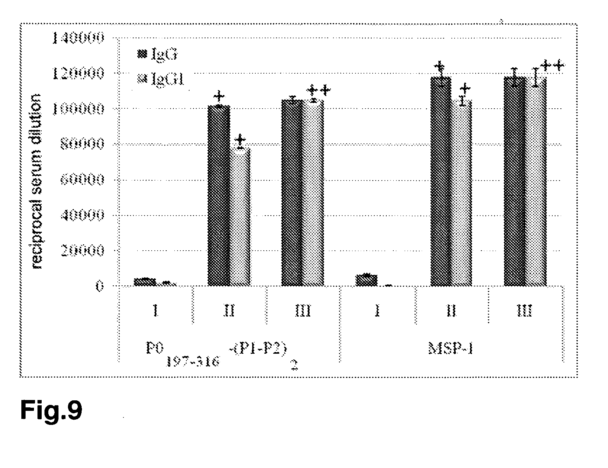

Fig. 9 shows the level of IgG and IgG1 - class antibodies in the serum of mice immunized with the P protein complex and MSP-1 protein. Plus sign indicates statistically significant differences in comparison to the immunization I; Double plus sign denotes statistically significant differences in comparison to the immunization II;

The entire invention has been presented, using the examples described below.

### Example 1. Preparation of the pentameric P protein complex of Plasmodium falciparum

### 1.1 Preparation of the P protein complex

Expression of the P proteins complex according to the invention was carried out in a heterological bacterial system, using *Escherichia coli.* The expression was performed in culture flasks on a LB medium at 37°C, containing 0.5% yeast extract, 1% pepton and 1% NaCl. The medium was supplemented with antibiotic - ampicillin (100 µg/ml). Each flask, containing media, was inoculated with the appropriate amount of the pre-cultured overnight *inoculum* of *E. coli* strain BL21 (DE3) bearing a vector containing the expression cassette according to the invention as defined above. The culture was carried out at 37°C, with intensive aeration and the optical density of the culture was monitored at 600 nm (OD₆₀₀). After one hour since the inoculation, ampicillin was added to the culture (100 µg/ml). By the time the optical density of the culture reached OD₆₀₀=0.8, the expression inductor - isopropyl β-D-1-thiogalactopyranoside (IPTG), was added up to the final concentration of 0.5 mM. The culture was continued for 4 hours, followed by the centrifugation of the cells (10 min., 3000x rpm), using Beckman preparative centrifuge. The cells were washed with a cold phosphate buffered saline (PBS) and the centrifugation was repeated. The cells were then desintegrated by sonication (45 s per cycle with 60 s interrupt), using a sonicator equipped with a tytanium probe (diameter, 2 cm; MSE ). 20-Desintegration cycles were performed. Throughout the desintegration the temperature (+4 °C) was monitored and the pH was maintained at approx. 7. The resulting lysate of bacterial cells was fractionated by centrifugation (20 min., 12000x rpm, at 4°C) and the soluble fraction of the cytoplasmic proteins combined with the ribosomal proteins was obtained. Ribosomes were sedimented by addition of a Triton X-100 detergent to this soluble fraction (up to a final concentration of 1 %) using a magnetic stirrer for 60 min. at 4 °C and followed by ultracentrifugation (90 min., 38000x rpm, Beckman rotor TY50.2Ti). The obtained supernatant, named herein an S-100 fraction, contained the cytoplasmic proteins fraction, containing among others a pentameric complex of P proteins.

To isolate and purify the P proteins, the fraction S-100 was mixed with GST-trap bead and subjected to affinity chromatography. Binding of the pentameric complex to a chromatographic bead was carried at 4 °C for 60 min, followed by centrifugation (10 min., 300 rpm; preparative Beckman centrifuge) that allowed for separation of the non-binding proteins present in the fraction S-100. Following the centrifugation, the sedimented beads were washed with a 1 0-fold volume of PBS and subsequently it was washed with an equal volume of PBST (phosphate buffered saline containing 0.1% Tween 20). Finally, to remove the S-1 00 fraction proteins that might have bound in a non-specific way to the GST-trap, the beads were washed with 2-fold volume of PBS. To release the pentameric complex of P proteins, the GST-trap beads were incubated for 60 min. at 37°C with the human thrombine solution (22 units/ml of bead). Subsequently, the thrombine inhibitor, namely phenylmethanesulphonylfluoride, PMSF; final concentration, 1 mM) was added to the obtained supernatant, containing the P proteins complex as defined according to the invention. The herein described procedure allowed for the isolation of the homogeneous pentameric P proteins complex.

### 1.2 Analysis of the purified P protein complex

The following samples were prepared and analysed: proteins (30 µg) of the cell extract of *E.coli* transformed with the vector, bearing expression cassette, prior to (-IPTG) and after (+IPTG) induction; proteins (30 µg) of the inclusion bodies, constituting the fraction of insoluble proteins; proteins (30 µg) of the S-100 fraction, containing the soluble proteins; proteins (5 µg) of the P proteins complex, with the GST label bound to P0 protein; proteins (5 µg) of the P proteins complex, with the GST label removed; (Fig. 3).

### Example 2. Analysis of the properties of the P protein complex, P0(₁₉₇₋₃₁₆)-(P1-P2)₂

The protein sample obtained according to the Example 1 was subjected to the following biochemical analysis:

### 2.1 Analysis employing size exclusion chromatography

The obtained P proteins complex was subjected to size exclusion chromatography using Superose 12 HR 10/30, equilibrated with a Tris-HCl buffer (20 mM, pH 7.5, containing 150 mM NaCl) using the FPLC Akta Purifier system. Figure 4. Analysis employing size exclusion chromatography.
The P proteins complex was eluted as a single symmetrical peak, indicating that the preparation is homogeneous. This single peak corresponds to a purified pentameric protein complex: P0(₁₉₇₋₃₁₆)-(P1-P2)₂

### 2.2 Mass spectrometry analysis

To determine the precise stoichiometry of the P protein complex obtained according to the example 1, a mass spectrometry analysis performed under non-denaturating conditions was carried out.

The molecular mass of the complex determined in this experiment was estimated as 63286 Da and corresponds to the theoretical mass of the P protein complex calculated as 63118 according to the invention. The analysis revealed that P protein complex comprises five polypeptides, including a P0(₁₉₇₋₃₁₆) protein fragment (13196 Da) and two dimers P1-P2 (13013 Da and 11948 Da, respectively).

### Example 3. Immunological analysis of the pentameric P protein complex P0(₁₉₇₋₃₁₆)-(P1-P2)₂

### 3.1 Immunization of animals

The immunostimulating properties of the pentameric P protein complex were assessed on mice (6-7 weeks old, weight 18 g +/- 2). Mice were obtained from the Institute of Experimental and Clinical medicine, Warsaw, Poland. Each group of mice (including control group) consisted of 18 animals. The analysed complex was combined with complete (the first immunization) and incomplete (the second and the third immunization) Freund adjuvant in a 1:1 volume ratio. Mice were immunized by intramuscular injection of a sterile suspension (100 µl) containing 50 µg of protein at days 0, 21 and 42. Blood samples were collected in two weeks intervals following the immunizations, that is at days 14, 35 and 56 (for both experimental and control groups). A control groups received only a complete, followed by incomplete, Freund adjuvant (group I) and PBS, phosphate buffered saline (group II), respectively. Additionally, the samples were also taken from non-immunized mice one week after they were delivered to the animal facility. Serum was then used for the determination of the class of antibodies produced, and lymphocytes isolated from blood and spleen were used for the analysis of toxicity of proteins, cell proliferation and determination of cell surface receptors.

A known antygen of *P. falciparum -* MSP-1 protein - was used as a reference in immunological analysis. In a control analysis the MSP-1₁₉ protein was used (96 amino acids, position of amino acids in a complete protein: 1526-1622; SWISS-PROT, MSP1 PLAFW, accession number P04933). This protein was obtained by cloning and heterologically expressed using *E. coli* strain, followed by purification to homogeneity. The reference antygen prepared in such a way was used in the immunological analysis.

### 3.2 Analysis of the toxicity of P proteins

Murine spleen lymphocytes were incubated for 24-48 h with the investigated P protein complex. P protein complex concentration ranged from 10 to 1000 µg/ml. At a defined time, that is after 24 h and 48 h, viability of the cells was determined by the MTT reduction method. The results have been presented on Figure 6, showing the correlation between the survival of the cells (expressed as %) and the concentration of the investigated protein.
The investigated protein complex did not show the toxic effect on lymphocytes within the 10-200 µg/ml concentration range. A similar behaviour was observed for the MSP-1 protein, however, when the incubation time with this antigen was extended to 48 h, viability of the cells was diminished by almost 35%.

### 3.3 Analysis of the lymphocytes proliferation induced by the P protein complex and MSP-1.

The lymphocytes were isolated from murine spleens of control and immunized (after the third immunization) animals. The lymphocyte proliferation analysis was carried out according to the standard procedures, and both MTT and ELISA BrdU tests yielded comparable results.

When compared with the control groups, lymphocytes of mice immunized with P protein complex and MSP-1 protein showed higher proliferation. The most significant increase of lymphocyte proliferation was observed for the MSP-1 reference protein (300 % increase, comparing to the control group) and for the P protein complex the lymphocyte proliferation increased by approximately 250%.

### 3.4 Analysis of the level of antibodies of IgM, IgG, IgG1 and IgG2a-class in the sera

The levels antibodies in the sera, collected two weeks after the immunizations, were determined using ELISA test. Plates were coated with the antigen as defined according to this invention, and with the MSP-1 reference protein. The results have been presented as a reciprocal of the highest dilution of the investigated serum for which the OD₄₀₅ nm equalled 0.1. All the investigated proteins induced the humoral response and in a manner that showed the statistically significant increase with the subsequent immunizations.

### 3.4.1 Comparison of the titer of IgM and IgG2a-class antibody obtained by immunization of mice with the P protein complex preparation and MSP-1 protein.

The results (below) have been presented as a reciprocal of the highest dilution of the investigated serum for which the OD₄₀₅ nm equalled 0.1. The Roman numerals I, II and III denote subsequent immunizations.

As indicated by the data (Figure 8) the investigated proteins induced the humoral response that was increasing significantly with subsequent immunizations. (except for IgM). The most effective induction of IgM-class antibodies took place in the presence of the MSP-1 reference protein, and especially after the immunization I with the subsequent decline of the antibody titer. Unlike the MSP-1 protein, the P protein complex induced the production of the immunoglobullins of IgM-class at the very low level. This level did not change or was slightly lowered with the subsequent immunizations. The most noticeable increase of the titers of IgG2a-class antibodies was observed after the immunization II. The effects of MSP-1 protein and P protein complex were similar (the presence of IgG2a was detected at the 4800-fold and 10000-fold serum dilution, respectivly). After the immunization III the IgG2a titer levelled out for both P protein complex and MSP-1 protein, at the dilution within the 13000-fold to 20000-fold range.

### 3.4.2 Comparison of the titers of IgG and IgG1-class antibody obtained by immunization of mice with the P protein complex preparation and MSP-1.

IgG Antibodies constitute the most important immunoglobulins of the secondary response. They show high affinity to an antigen together with the opsonization capabilities (initiation of the "killing" mechanisms) and are able to activate complement and penetrate the placenta. As the presence of IgG is detected after certain amount of time following contact with the antigen, therefore in the investigation very low titers of this antibodies were observed following the first immunization, however the titer increased substantially after the second and the third immunizations.

The results (below on Fig.9) have been presented as a reciprocal of the highest dilution of the investigated serum for which the OD₄₀₅ nm equalled 0.1. The Roman numerals I, II and III denote subsequent immunizations.

The MSP-1 protein and P protein complex induced very good the synthesis of antibodies (the presence of IgG was detected at the 118000-fold and 100000-fold serum dilution, respectively). After the immunization III the IgG titer levelled out for both P protein complex and MSP-1 protein, at the dilution within the 100000-fold to 118000-fold range. Considering the IgG1 subclass antibodies the MSP-1 protein was the most effective at inducing the synthesis of IgG1 antibodies (104000-fold serum dilution) and the P protein complex (78000-fold serum dilution) was the second best. After the immunization III the IgG1 titer increased and the levels of both investigated antigens were approximately the same.

### 3.5 Comparison of the cytotoxic (Tc) and regulatory (Treg) lymphocytes counts induced by immunization of mice with the P protein complex preparation and MSP-1.

The Tc lymphocytes are the most important cells involved in the immunological response during the pre-erythrocytic *Plasmodium falciparum,* that destroy hepatocytes infected by the parasite. The Treg lymphocytes inhibit the inflammatory response, however their presence is beneficial for the host at the later stage of the infection, when it prevents further pathological consequences. Had the suppresive effect of these cells appeared too early during infection, it would allow for an uncontrolled increase of the number of parasites and effectively making the serious form of malaria more probable. The lymphocyte Tc and Treg population counts (expressed as %) were determined 2 weeks after each immunization and the surface markers (CD4-CD8+ and CD4+CD25+, respectively) characteristic for these cells were determined using flow cytometry. The results have been presented in the table (Table 1, below).

**Table 1**

| | K1 | | P0₁₉₇₋₃₁₆-(P1-P2)₂ | | MSP-1 | |
|---|---|---|---|---|---|---|
| immunizacja | % Tc | % Treg | % Tc | % Treg | % Tc | % Treg |
| I | 6,33±0,5 | 24,4±0,9 | 6,97±0,4 | 21,2±2,3 | 7,03±0,9 | 23,0±2,6 |
| II | 6,79±1,2 | 24,0±2,8 | **10,9±0,02⁺** | 20,1 ±02 | **12,3±1,2⁺** | 23,4±1,1 |
| III | 8,19±0,5 | 23,4±0,5 | 5,9±1,3 | **35,7±3,3⁺⁺** | 6,56±0,7 | **33,8±6,2⁺⁺** |

| | | | | | | |
|---|---|---|---|---|---|---|
| Plus sign indicates statistically significant differences in comparison to the immunization I; Double plus sign denotes statistically significant differences in comparison to the immunization II; | | | | | | |

The data presented in Table 1 ensures that the investigated proteins were able to induce the formation of populations of Tc lymphocytes (predominantly observable after the immunization II) and Treg lymphocytes (after the immunization III) and the induction levels for both Tc and Treg lymphocytes were comparable. It is worth noting that the delayed emergence of Treg lymphocytes is beneficial regarding the on-set of immunity.

On the basis of the performed immunological studies it can be concluded that the P protein complex P0(₁₉₇₋₃₁₆)-(P1-P2)₂ can effectively induce the immunological response. Moreover, the course and inhibition of the inflammatory reactions is comparable to these of the MSP-1 reference protein. This provides the rationale for the claim, that the P proteins complex P0(₁₉₇₋₃₁₆)-(P1-P2)₂ can be employed for the preparation of a vaccine against malaria.

### Example 4. Construction of the expression cassette

In order to obtain the expression cassette, the following nucleotide sequences deposited in the database of *Plasmodium falciparum* 3D7 genome (www: http://www.genedb.org/Homepage/Pfalciparum) were used. The genes for the proteins: P0 (PF11_0313), P1 (PF11_0043) and P2 (PFC0400w) were synthesized by the GenScript company. Subsequently, they were amplified using the PCR method and were cloned into the bacterial vector pGEX4T-1.

The cloning began with a gene corresponding to the fragment of P0 protein (comprising codons for the amino-acids in the range 197-316) that was cloned into the pGEX4T-1 vector, employing the unique restriction sites within this vector, that is BamHI and EcoRI. Then the genes for ribosomal proteins P1 and P2 were cloned-in, using unique pairs of restriction sites for enzymes EcoRI/SaII and Sall/Notl. The above-mentioned genetic manipulations were performed according to the standard genetic procedures. The RBS regulatory sequences and the linker sequences, that separated individual genes, were introduced by cloning with the nucleotide starters used for DNA amplification by PCR method. The expression cassette has been presented in Sequence 2.

The expression cassette contains:
- the nucleotide sequence, coding the GST protein fragment fused with DNA encoding P0 protein fragment (residues 197-316) of *Plasmodium falciparum*; between the DNA sequence for GST protein and the DNA fragment for P0 polipeptide there is a sequence, coding the amino acids that form the cleavage site for the proteolytic enzyme, thrombine;
- the linker sequence together with the ribosome binding sequence (RBS);
- the nucleotide sequence, coding the complete protein P1 of *Plasmodium falciparum*;
- the linker sequence together with the ribosome binding sequence (RBS);
- the nucleotide sequence, coding the complete protein P2 of *Plasmodium falciparum*.

### The list of sequences

The amino acid sequence of the pentameric P protein complex of *Plasmodium falciparum* P0(₁₉₇₋₃₁₆)-(P1-P2)₂
Sequence 1
   - P0₁₉₇₋₃₁₆ 
GSMVIYDAKVLDITDEDILEKFSKGVSNVAALSRATGVITEASYPHVFVEAFKNIVALIIDS
DYTFPLMENIKKMVENPEAFAAVAAPASAAKADEPKKEEAKKVEEEEEEEDGFMGFGMFD
   - P1 
MASIPASELPECEKQELLCTYAALILHEEKMSITSDNILKLIKNSNNTVLPYLPMLFERALK
GKDIQSLLSNLSVGSAPAAAAQVTTEKPSEDKKEAKKEEKVEEEEEEDDLGFSLFG
   - P2 
MAMKYVAAYLMCVLGGNENPSTKEVKNVLGAVNADVEDEVLNNFIDSLKGKSCHELITDGLK
KLQNIGGGVAAAPAGAAAVETAEAKKEDKKEEKKEEEEEEEDDLGFSLFG
Sequence 2
   Reading frames of GST, P0₁₉₇₋₃₁₆, P1 and P2 are marked with a double line; DNA sequence coding the amino acids that form the cleavage site for the proteolytic enzyme, thrombine is marked with a single line;
   Restriction sites for the enzymes EcoRI, Sall and Notl is marked with a dashed line; The linker sequences are shadowed and the ribosome binding sequence (RBS) of the linker sequence is shaded.

ATGTCCCCTATACTAGGTTATTGGAAAATTAAGGGCCTTGTGCAACCCACTCGACTTCTTTT
GGAATATCTTGAAGAAAAATATGAAGAGCATTTGTATGAGCGCGATGAAGGTGATAAATGGC
GAAACAAAAAGTTTGAATTGGGTTTGGAGTTTCCCAATCTTCCTTATTATATTGATGGTGAT
GTTAAATTAACACAGTCTATGGCCATCATACGTTATATAGCTGACAAGCACAACATGTTGGG
TGGTTGTCCAAAAGAGCGTGCAGAGATTTCAATGCTTGAAGGAGCGGTTTTGGATATTAGAT
ACGGTGTTTCGAGAATTGCATATAGTAAAGACTTTGAAACTCTCAAAGTTGATTTTCTTAGC
AAGCTACCTGAAATGCTGAAAATGTTCGAAGATCGTTTATGTCATAAAACATATTTAAATGG
TGATCATGTAACCCATCCTGACTTCATGTTGTATGACGCTCTTGATGTTGTTTTATACATGG
ACCCAATGTGCCTGGATGCGTTCCCAAAATTAGTTTGTTTTAAAAAACGTATTGAAGCTATC
CCACAAATTGATAAGTACTTGAAATCCAGCAAGTATATAGCATGGCCTTTGCAGGGCTGGCA
AGCCACGTTTGGTGGTGGCGACCATCCTCCAAAATCGGATCTGGTTCCGCGTGGATCCATGG
TAATTTATGATGCAAAAGTTTTAGATATTACCGATGAAGATATCTTAGAAAAATTTCAAAA
GGTGTATCTAATGTCGCTGCTTTATCTAGAGCTACAGGTGTTATTACAGAGGCTTCTTATCC
ACATGTATTTGTCGAAGCATTCAAGAATATTGTTGCTTTAATTATAGATTCAGATTATACTT

TCCCATTAATGGAAAATATTAAAAAAATGGTTGAAAATCCAGAAGCATTTGCCGCAGTTGCT
GCACCTGCATCTGCAGCCAAAGCTGATGAACCCAAAAAAGAAGAAGCCAAAAAAGTAGAAGA
GGAGGAAGAAGAAGAAGAAGATGGATTCATGGATTTGGAATGTTTGATTAAGAATTCAGGA
GTACAAAAATGGCATCAATTCCAGCATCAGAATTACCAGAATGTGAAAAGCAGGAACTTTTA
TGTACCTATGCTGCTTTAATATTACATGAAGAAAAAATGAGTATAACAAGTGACAATATTTT
AAAGCTTATTAAAAATTCTAACAATACTGTTTTGCCATACTTACCAATGCTTTTCGAAAGGG
CTTTAAAGGGAAAAGATATTCAGAGCTTATTAAGTAACTTAAGTGTAGGAAGTGCCCCTGCA
GCTGCTGCCCAAGTTACAACTGAGAAACCAAGTGAAGACAAAAAGGAAGCCAAAAAAGAAGA
GAAAGTTGAGGAAGAAGAAGAAGAAGATGATTTAGGTTTCTCCTTATTTGGTTAAGTCGACA
GGAGTACAAAAATGGCTATGAAATACGTTGCTGCATATCTTATGTGTGTATTGGGAGGAAAT
GAAAACCCAAGCACAAAAGAAGTTAAGAATGTGTTAGGAGCCGTAAATGCTGATGTAGAAGA
TGAAGTTTTAAACAATTTTATTGATTCATTAAAAGGAAAGAGTTGCCATGAATTAATTACTG
ATGGATTAAAAAAATTACAAAATATTGGAGGTGGTGTAGCTGCCGCACCAGCTGGTGCCGCT
GCAGTAGAAACTGCTGAAGCTAAGAAAGAAGATAAGAAAGAAGAAAAGAAAGAAGAAGAAGA
GGAAGAAGAAGACGACTTAGGATTTCCTTATTTGGTTAAGCGGCCGC

## Claims

1. A ribosomal P protein complex from Plasmodium falciparum, containing amino acid sequence fragment of the P0 protein including amino acid residues from 197 to 316 and the amino acid sequences of two complete P1 and P2 proteins defined as "sequence 1" and shown in Fig. 1.

2. The antigen according to clam 1, **characterized in that** the amino acid sequences of P1 and P2 proteins are preferably in heterodimeric system.

3. The antigen of malaria pathogen according to claim 1 and 2 for use in obtaining the vaccine against malaria.

4. The method to obtain the antigen according to claim 1, comprising the method of protein overexpression with the use of the expression vector **characterized in that** said expression vector uses said expression cassette of "sequence 2" containing three genes in polycistronic system encoding P protein complex according to claim 1 and 2.

5. The expression cassette as the component of the expression vector used according to the method of claim 4, **characterized in that** the expressed sequence is the three gene in polycistronic system, of which every gene contains the separate regulatory RBS sequences and the linker sequences and the expression cassette is defined as "sequence 2" and shown in Fig. 2.

6. The expression casette according to claim 5 **characterized in that** the GST protein is preferably fused to the N-terminal end of the P0 fragment protein.

## Patentansprüche

1. Ribosomaler P-Proteinkomplex aus Plasmodium falciparum enthaltend ein Aminosäuresequenzfragment des P0-Proteins, welches die Aminosäurereste von 197 bis 316 umfasst sowie Aminosäuresequenzen von zwei Vollproteinen P1 und P2, bezeichnet als "Sequenz 1" und dargestellt auf der Fig.1.

2. Das Antigen nach Anspruch 1 **dadurch gekennzeichnet, dass** die Aminosäuresequenzen der P1- und P2-Proteine günstig im heterodimerischen System auftreten.

3. Das Antigen des Malariapathogen nach Ansprüchen 1 und 2 verwendet bei Gewinnung des Malariaimpfstoffs.

4. Das Gewinnungsverfahren des im Anspruch 1 bezeichneten Antigens, das die Methoden der Proteinüberexpression unter Anwendung des Expressionsvektors umfasst, **dadurch gekennzeichnet, dass** im Expressionsvektor eine Expressionskassette der "Sequenz 2", welche ein den P-Proteinkomplex nach Ansprüchen 1 und 2 kodierendes polycistronisches System von drei Genen enthält, benutzt wird.

5. Die Expressionskassette als Bestandteil des Expressionsvektors, der nach dem im Anspruch 4 definierten Verfahren benutzt wird **dadurch gekennzeichnet, dass** die der Expression unterliegende Sequenz das polycistronische System von drei Genen bildet, wobei jedes von ihnen isolierte RBS-Regulatorsequenzen sowie Verbindersequenzen enthält, und die Expressionskassette als "Sequenz 2" geschildert und auf der Fig. 2 gezeigt wird.

6. Die Expressionskassette nach Anspruch 5 **dadurch gekennzeichnet, dass** das an das N-terminale Ende des fragmentarischen P0-Proteins fusionierte Protein GST günstig auftritt.

## Revendications

1. Un complexe des protéines P ribosomale de Plasmodium falciparum, contenant du fragment de séquence d'acide aminés de la protéine P0 comprenant les résidus d'acides aminés à partir de 197 à 316 et les séquences d'acides aminés des deux protéines P1 et P2 complète définié comme "séquence 1" et indiqué sur la Fig. 1.

2. L'antigne selon la revendication 1, **caractérisée en ce que** séquences de l'acide aminé de protéines P1 et P2 sont, de préférence, dans le système hétérodimère.

3. L'antigène de l'agent pathogène du paludisme selon la revendication 1 et 2 destiné à être utilisé pour obtenir du vaccin contre le paludisme.

4. Le procédé pour obtenir l'antigne selon la revendication 1, comprenant le procédé de la surexpression de la protéine à l'aide du vecteur d'expression, **caractérisé en ce que** ledit vecteur d'expression utilise ladite cassette d'expression de "séquence 2" contenant trois gènes dans polycistronique système codant complexe de protéine P selon la revendication 1 et 2.

5. La cassette d'expression en tant que composant du vecteur d'expression utilisé selon le procédé de la revendication 4, **caractérisé en ce que** la séquence est les trois gène système polycistronique, dont chaque gène contient les séquences de régulation distincts RBS et les séquences de liaison, et l'cassette d'expression est définie comme "Séquence 2" et indiqué sur la Fig. 2.

6. La cassette d'expression selon la revendication 5, **caractérisé en ce que** la protéine GST est de préférence fusionné à l'extrémité de N-terminale de la protéine P0 de fragment.
